# EUROPÄISCHE PATENTSCHRIFT

(11) **EP 0 648 738 B1**
(45) Veröffentlichungstag und Bekanntmachung des Hinweises auf die Patenterteilung: **08.01.1997**
(21) Anmeldenummer: 94116234.9
(22) Anmeldetag: 14.10.1994
(51) Int. Cl.: C07C 231/04, C07C 235/80

(54) **Verfahren zur Herstellung von Acetoacetarylamiden**
Process for the preparation of acetoacetarylamides
Procédé pour la préparation d'acéto-acétamides N-aryliques

(30) Priorität: 19.10.1993 DE 4335613
(43) Veröffentlichungstag der Anmeldung: 19.04.1995
(73) Patentinhaber: HOECHST AKTIENGESELLSCHAFT, 65929 Frankfurt am Main (DE)
(72) Erfinder: Mack, Karl Ernst, Dr., D-65207 Wiesbaden (DE); Bohusch, Michael, Dr., D-61267 Neu Anspach (DE)

(56) Entgegenhaltungen:
- DE-A- 1 518 881
- DE-A- 2 647 499
- FR-A- 2 309 514
- DATABASE WPI Week 8237, Derwent Publications Ltd., London, GB; AN 82-77578E & JP-A-57 126 453 (DAINIPPON) 6. August 1982
- ULLMANN, 1990, Vol A15, SEITE 71

## Beschreibung

Acetoacetarylamide sind in der Farbenindustrie bedeutende Ausgangsmaterialien für die Herstellung von Pigmenten, deren Farbton und Qualität in hohem Maße vom Reingehalt und dessen Schwankungen bei den Acetoacetarylamiden abhängen. Es besteht daher ein großes Interesse an Acetoacetarylamiden besonders hoher und gleichbleibender Reinheit.

In DE-OS 1518881 wird die kontinuierliche Herstellung von Acetoacetarylamiden aus Diketen und Arylamin in Wasser als Reaktionsmedium bei Temperaturen von 0 bis 50°C beschrieben. Wegen der Schwerlichkeit der Einsatz- und der Endprodukte in Wasser besteht jedoch trotz empfohlenem heftigem Rühren bei der Umsetzung die große Gefahr der ungleichmäßigen Umsetzung und Verunreinigung der Produkte durch Einschluß von unumgesetztem Diketen oder Arylamin in einzelne sich bildende Produktpartikel. Die Begrenzung der Temperatur auf 50°C wegen der Gefahr der Hydrolyse von Diketen läßt selbst bei diesen erhöhten Temperaturen kaum nennenswerte Löslichkeitseffekte erwarten und beschränkt außerdem die Umsetzungsgeschwindigkeit von Arylamin und Diketen derart, daß Reaktionszeiten von 20 bis 90 Minuten erforderlich sind. Die langen Reaktionszeiten und die technisch aufwendige Handhabung von Suspensionen behinderen die optimale Nutzung dieses kontinuierlichen Verfahrens erheblich.

In einer Reihe von Patentschriften werden zur Verbesserung der Löslichkeitsverhältnisse organische Lösemittel vorgeschlagen, wie z.B. Toluol oder Xylol in DOS 2749327 oder wie Alkohole in der japanischen Offenlegungsschrift 57-126 453. Diese Lösemittel weisen jedoch häufig so gute Lösevermögen für die Acetoacetarylamide auf, daß hohe Ausbeuten z.B. erst nach aufwendigem Verdampfen der Lösemittel unter gleichzeitigem Mitfällen von Verunreinigungen erzielt werden können oder aber wie im Falle der Alkohole die Reaktionsprodukte durch Zugabe von Wasser nach der Reaktion durch Fällen isoliert werden müssen. Darüber hinaus wird insbesondere bei der japanischen Offenlegung eine kontinuierliche Verfahrensweise nicht erwähnt, wohl wegen der ebenfalls begrenzten Reaktionstemperatur von 60°C und der damit verbundenen langen Reaktionszeit von allgemein über 60 min.

In Anbetracht dieser Einschränkungen und Nachteile bestand ein großes Bedürfnis nach einem verbesserten Verfahren, durch das die den bekannten Verfahren innewohnenden Nachteile vermieden und gute bis sehr gute Ausbeuten, ein hoher Reinheitsgrad und verkürzte Reaktionszeiten ermöglicht werden. Ein kontinuierliches Verfahren wird aufgrund seiner hohen Raum-Zeit-Leistung (hergestellte Produktmenge pro Zeiteinheit und Reaktorvolumen) wirtschaftliche und aufgrund von kleinen Apparateabmessungen mit dementsprechend geringen Rückhaltevolumen bei hochreaktiven Einsatzstoffen sicherheitstechnische Vorteile bieten.

Diese Aufgabe wird gelöst durch ein Verfahren zur Herstellung von Acetoacetarylamiden der Formel I 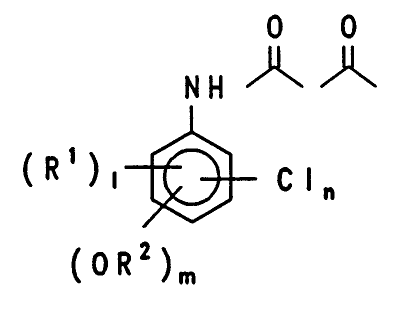
worin
- R¹, R²: gleiche oder verschiedene Alkylreste
- l, m: = 0, 1 oder 2
- n: = 0 oder 1
sind,
durch Addition von Diketen an das entsprechende Arylamin, indem man das Arylamin in Gegenwart eines Gemisches aus Wasser und eines (C₁-C₄)-Alkanols innerhalb von 0,1 bis 10 min. bei Temperaturen von 60°C bis 100°C kontinuierlich mit Diketen umsetzt.

Die verwendeten Arylamine können bis zu jeweils zwei Alkyl- oder Alkoxygruppen tragen. Interessant ist die Umsetzung von Alkyl- bzw. Alkoxyverbindungen, die im Alkylrest jeweils bis zu drei C-Atome enthalten und Methyl, Ethyl, n-Propyl oder i-Propyl darstellen. Von besonderer Bedeutung ist die Umsetzung von Anilin, 2-Methylanilin, 2,4-Dimethoxyanilin, 4-Isopropylanilin, 2-Methoxyanilin, 4-Ethoxyanilin, 2,4-Dimethylanilin, 2,5-Dimethoxyanilin, 2-Chloranilin und 4-Chlor-2,5-Dimethoxyanilin.

Die Amine können in technischer Qualität eingesetzt werden; feste Amine können wasserfeucht sein.

Diketen kann ebenfalls in technischer Qualität eingesetzt werden. Es hat sich als vorteilhaft erwiesen im Überschuß von 3 bis 30 Mol-%, insbesondere 5 bis 25 Mol-%, zu arbeiten.

Als Alkohol können Methanol, Ethanol, n-Propanol, i-Propanol und die Butanole eingesetzt werden. Es hat sich in vielen Fällen bewährt, die mit Wasser vollständig mischbaren Alkohole wie Methanol, Ethanol und die Propanole zu verwenden. Aus gewerbehygienischen Gründen wird Ethanol bevorzugt.

Der Alkohol wird stets im Gemisch mit Wasser eingesetzt. Dabei stehen das Verhältnis von Alkohol zu Wasser, die Menge dieses Gemischs bezogen auf die einzelnen Arylamine und die maximale Reaktionstemperatur, d.h. der Siedepunkt des Alkohol/Wasser-Gemischs in komplexer Beziehung zueinander.

Der Wassergehalt im Alkohol/Wasser-Gemisch und dessen Menge bzgl. des Arylamins werden aus Gründen der Produktreinheit durch die Forderung bestimmt, daß einerseits die - gegebenenfalls heiße - Lösung eine möglichst hohe Konzentration aufweisen soll, andererseits das nach der Umsetzung entstandene Acetoacetarylamid bei Reaktionstemperatur noch gelöst bleiben soll. Allgemein sind solche Alkohol/Wasser-Gemische geeignet, die sich durch ihre Siedetemperatur von etwa 70 bis 100°C charakterisieren lassen. Beim Einsatz von Ethanol beträgt der Siedepunkt des Gemischs zweckmäßigerweise etwa 80 bis 90°C entsprechend einem Wassergehalt von etwa 20 bis 80 Gew.-%. Bewährt hat sich z.B. beim Einsatz flüssiger Arylamine wie dem des 2,4-Dimethylanilins ein Gehalt von etwa 70 Gew.-% Wasser, bei festen Arylaminen wie 4-Chlor-2,5-Dimethoxyanilin ein Gehalt von ca. 50 Gew.-% Wasser.

Das Gewichtsverhältnis von Amin zu Alkohol/Wasser-Gemisch kann allgemein im Bereich von 1:1 bis 1:20 schwanken. Bewährt hat sich ein Bereich von etwa 1:1 bis 1:15, insbesondere 1:1 bis 1:10.

Die Umsetzungstemperatur soll mindestens 60°C betragen, um ausreichend rasche Umsetzungen zu ermöglichen; als günstiger erweist sich jedoch allgemein 70°C. Wegen der Instabilität des Diketens und wegen der Siedetemperatur von Wasser soll 100°C als maximale Reaktionstemperatur nicht überschritten werden; aus sicherheitstechnischen Gründen ist eine maximale Temperatur von 95°C vorzuziehen. Die optimale Reaktionstemperatur für die einzelnen Arylamine ist von deren Reaktivität maßgeblich abhängig und ist für jedes Amin durch Versuche zu ermitteln. Innerhalb des zweckmäßigen Temperaturbereichs von 70 bis 95°C erweist sich der Bereich von 75 bis 90°C als vorteilhaft; bei Verwendung von Ethanol mit 50 bis 70 Gew.-% Wasser sind Temperaturen von 83 bis 86°C günstig.

Die Einstellung und Begrenzung der Temperatur der stark exothermen Umsetzungen erfolgt nach der Startphase unter Eigenerwärmung - gegebenenfalls aber auch unter äußerer Wärmezufuhr - anschließend entweder durch Kühlen des Umsetzungsgemischs mittels äußerer Kühlung, durch Vorkühlen der Einsatzströme oder durch Erhöhen der Menge des Alkohol/Wasser-Gemischs in dem Maße, daß deren Wärmekapazität die freiwerdende Reaktionswärme bis zur gewünschten Temperatur aufnehmen kann.

Ein Vorteil des neuen Verfahrens ist es, die Umsetzung bei Siedetemperatur des Alkohol/Wasser-Gemischs durchzuführen und die Kühlung durch Kondensieren der Dämpfe von Alkohol und Wasser zu bewerkstelligen. Auf diese Weise wird eine technisch einfache, sichere und unter exakter Begrenzung der Temperatur wirksame Kühlung ermöglicht.

Die kontinuierliche Umsetzung von Diketen und Arylamin erfolgt unter möglichst kurzer Temperaturbelastung wobei als Umsetzungsdauer die durchschnittliche mittlere Verweilzeit im Reaktor betrachtet wird. Diese soll unterhalb von etwa 10 min. liegen. Bei ausreichend wirksamer Wärmeabfuhr z.B. durch Verdampfungskühlung können erheblich kürzere Verweilzeiten von z.B. etwa 0,5 bis 5 min. erzielt werden. Als günstig erweist sich bei den Umsetzungen z.B. von 2,4-Dimethylanilin, von 2-Methoxyanilin oder von 4-Chlor-2,5-Dimethoxyanilin in Anwesenheit von Ethanol mit 70 bzw. 50 Gew.-% Wasser eine Umsetzungsdauer von 0,75 bis 3 Minuten, insbesondere von etwa einer Minute.

Trotz Optimierung der Einsatzverhältnisse verbleibt nach der Isolierung des Acetoacetarylamids ein Teil des Produkts entsprechend seiner Löslichkeit in der Mutterlauge. Die hochselektiv verlaufende Umsetzung gemäß des neuen Verfahrens erlaubt es, ohne nennenswerten Qualitätsverlust der Produkte die Mutterlauge mehrmals zur Umsetzung zurückzuführen und auf diese Weise die sonst in der Mutterlauge verbleibende Menge an Acetoacetarylamid weitgehend zu gewinnen. Die Mutterlauge wird dabei durch ein frisches Alkohol/Wasser-Gemisch ergänzt, das mengenmäßig jenem Mutterlaugenanteil entspricht, der als Produktfeuchte im Rohprodukt nach der Isolierung verblieben ist und anschließend durch Waschen entfernt wird.

Als Reaktoren eignen sich Apparate, in denen einerseits eine intensive Vermischung der Einsatzkomponenten möglich ist, wie z.B. durch Rühren, durch Mischen in Pumpen oder durch statische Mischer oder auch Mehrstoffdüsen. Andererseits muß eine wirksame indirekte Kühlung oder Verdampfungskühlung gewährleistet sein. Geeignete Reaktoren sind z.B. Rührkessel oder Rührkesselkaskade, statische Mischer mit anschließendem Strömungsrohr oder eine als Mischaggregat dienende Kreiselpumpe - integriert in einen Umlaufverdampfer, Fallfilm- oder Dünnschichtverdampfer.

Die kontinuierliche Dosierung von Arylamin und Diketen in den Reaktor erfolgt stets getrennt, die des Alkohol/Wasser-Gemischs bzw. von rückgeführter Mutterlauge kann entweder separat oder zusammen mit dem Arylamin erfolgen. Der kontinuierliche Austrag des Reaktionsgemischs geschieht entweder per Pumpe oder einfacher per Überlauf mit Syphon.

Die Umsetzung erfolgt allgemein bei Normaldruck. Überdruck ist bei Anwendung der Verdampfungskühlung mit dem Ansteigen der Reaktionstemperatur verbunden und unter dem Sicherheitsaspekt nur begrenzt anwendbar. Das Arbeiten unter Vakuum kann in Kombination von besonderen Alkohol/Wasser-Gemischen aufgrund von Löslichkeitserfordernissen und im Zusammenhang mit einer zweckmäßigen Absenkung der Siedetemperatur vorteilhaft sein.

Katalysatoren sind bei der Umsetzung allgemein nicht erforderlich, können aber zur weiteren Beschleunigung der Reaktion in bekannter Weise verwendet werden. Hierzu zählen Protonsäure wie z.B. Essigsäure, Amine wie z.B. Triethylamin oder auch Ammoniumverbindungen. Andere Zusätze wie farbaufhellende Substanzen sind allgemein nicht erforderlich, obwohl es sich in besonderen Fällen, wie z.B. beim Einsatz fester Amine mit dunkler Lösefarbe als vorteilhaft erweist, geringe Mengen z.B. an Dithionit im Verlauf des Herstellungsprozesses hinzuzufügen.

Die Aufarbeitung des kontinuierlich aus dem Reaktor ausgeschleusten Produktstroms erfolgt in bekannter Weise durch Kühlen und Kristallisation der Acetoacetarylamide. Die Produkte fallen gut kristallisiert an und können wegen ihrer Grobkörnigkeit einfach und schnell durch Filtrieren isoliert werden. Die anfallende Mutterlauge kann allgemein bei der Synthese unter Verbesserung der Produktausbeute wiederverwendet werden.

Das anfallende Rohprodukt wird zur Entfernung von Mutterlauge z.B. mit Alkohol/Wasser gewaschen, das gereinigte Produkt bei Bedarf getrocknet oder durch schonende Destillation in einer wäßrigen Aufschlämmung von anhaftendem Alkohol befreit und als wasserfeuchtes Produkt isoliert.

Nach destillativer Rückgewinnung von Alkohol/Wasser verläuft das neue Verfahren abwasserfrei.

Vorteile des neuen Verfahrens sind die bei der Umsetzung aufgrund hoher Raum-Zeit-Leistungen bis zu 20 kg Produkt/l x Stunde erforderlichen kleinen Reaktoren und die damit verbundene erhöhte Arbeitssicherheit durch geringe Rückhaltevolumen an hochreaktivem Diketen. Weiterhin gelingt es, die Acetoacetarylamide als allgemein gut kristallisierte, leicht isolierbare Produkte in hoher, für das Einsatzgebiet Pigmente geeigneter Reinheit herzustellen. Darüber hinaus sichert die Rückführung von produktenthaltender Mutterlauge zur Synthese als bedeutendes Merkmal des neuen Verfahrens eine optimale Produktausbeute bis ca. 98 % (bzgl. Arylamin) und erlaubt durch einfache destillative Rückgewinnung von wäßrigem Alkohol aus Aufarbeitungsströmen eine abwasserfreie Durchführung des Verfahrens.

Die folgenden Beispiele sollen das Verfahren erläutern ohne es jedoch darauf zu beschränken.

### Beispiel 1

2,48 kg/h (20,5 mol/h) 2,4-Dimethylanilin, 1,83 kg/h (21,3 mol/h) Diketen (Reinheit 98 %) und 8 kg/h eines Gemisches aus 2,4 kg/h Ethanol und 5,6 kg/h Wasser werden getrennt aber gleichzeitig in einem auf ca. 80°C vorgewärmten Reaktor dosiert. Der Reaktor besteht aus einem doppelwandigen Glasgefäß mit ca. 0,6 l Volumen und ist mit einem Rührer, Thermometer, Rückflußkühler und unten mit einem Ablauf versehen. Unmittelbar nach Dosierungsbeginn kommt es im Reaktor aufgrund der Wärmeentwicklung der Reaktion bei ca. 85°C zum Sieden unter Rückfluß. Die Heizung des Reaktors wird unterbrochen und die Umsetzung fortgesetzt, wobei der Reaktorinhalt durch ständiges Abpumpen auf ca. 0,3 l Volumen begrenzt wird. Der Reaktoraustrag wird unter Rühren auf ca. 15°C abgekühlt und die entstandenen hellen Kristalle durch Filtrieren von ca. 7 kg/h hellgelbe Mutterlauge abgetrennt. Das mit ca. 5 kg/h anfallende feuchte Produkt wird mit ca. 4 kg/h kaltem wäßrigen 30 %igem Ethanol gewaschen und getrocknet. Es werden 4 kg/h (19,5 mol/h) an 2',4'-Dimethylacetoacetanilid (Schmp. um 89°C) erhalten, entsprechend einer Ausbeute von 95 % bzgl. Amin.

Zur Wiederverwendung der anfallenden Mutterlauge von ca. 7 kg/h wird diese durch Zugabe von ca. 1 kg/h wäßriges, 30 %iges Ethanol auf 8 kg/h ergänzt und in oben beschriebener Weise mit 2,4-Dimethylanilin und Diketen umgesetzt und zu 4,08 kg/h Produkt (Ausbeute 97 %) aufgearbeitet.

### Beispiel 2

2,48 kg/h 2,4-Dimethylanilin, 1,83 kg/h Diketen (Reinheit 98 %) und 9 kg/h wäßriges 30 %iges Ethanol werden getrennt aber gleichzeitig bei ca. 20°C in einem Reaktor dosiert, der aus einem statischen Mischer (ca. 0,5 ml; Firma Sulzer) und einem sich anschließenden Strömungsrohr mit ca. 0,2 l Volumen und einem Durchmesser von 6 mm besteht. Das Strömungsrohr enthält keine Einbauten, ist mit Außentemperaturfühlern versehen und durch Isoliermaterial wärmeisoliert. Aufgrund der Reaktionswärme stellt sich entlang des Strömungsrohres ein Temperaturprofil ein, das nach etwa der halben Rohrlänge ca. 78°C aufweist. Der Reaktoraustrag wird unter Rühren auf ca. 15°C abgekühlt und analog des Beispiels 1 aufgearbeitet. Die Ausbeute an 2',4'-Dimethylacetoacetanilid beträgt 93,5 % bzgl. des Amineinsatzes. Unter Wiederverwendung der anfallenden ca. 8 kg/h Mutterlauge, Ergänzung um ca. 1 kg/h mit wäßrigem 30 %igem Ethanol auf 9 kg/h und Umsetzung mit 2,4-Dimethylanilin und Diketen in oben beschriebener Weise wird die Ausbeute am 2',4'-Dimethylacetoacetanilid auf 96,5 % gesteigert ohne daß die Produktqualität (Schmp. um 89°C) abnimmt.

### Beispiel 3

2,4 kg/h (19,5 mol/h) 2-Methoxyanilin, 1,98 kg/h (23,1 mol/h) Diketen (Reinheit 98 %) und 6,9 kg/h wäßriges 30 %iges Ethanol werden getrennt aber gleichzeitig wie in Beispiel 1 beschrieben, umgesetzt. Der bei ca. 85°C, unter Rückfluß in einem durch Abpumpen konstant gehaltenen Volumen von ca. 0,3 l erzeugte Produktstrom wird auf ca. 15°C abgekühlt, das Reaktionsprodukt auskristallisiert und durch Filtrieren von 5,8 kg/h an Mutterlauge abgetrennt. Der helle Feststoff wird mit 5,8 kg/h wäßrigem 30 %igem Ethanol gewaschen und getrocknet. Es fallen 3,8 kg/h (18,3 mol/h) an 2'-Methoxyacetoacetanilid entsprechend 94 % Ausbeute bzgl. Amin an. Die Reinheit des Produkts beträgt über 99,5 %.

Zur Wiederverwendung der Mutterlauge wird diese mit 1,1 kg/h anfallender Waschlauge der Produktwäsche zu 6,9 kg/h ergänzt, mit Amin und Diketen im obigen Verhältnis umgesetzt. Ohne Qualitätsverlust wird die Ausbeute an 2'-Methoxyacetoacetanilid auf 97 % gesteigert.

### Beispiel 4

2,6 kg/h (24,3 mol/h) 2-Methylanilin, 2,4 kg/h (28 mol/h) Diketen (Reinheit 98 %) und 7,2 kg/h wäßriges 30 %iges Ethanol von dem man in nachfolgenden Reaktionscyclen 6 kg/h durch Mutterlauge ersetzt, werden getrennt aber gleichzeitig wie in Beispiel 1 zur Reaktion gebracht, wobei man das Reaktionsvolumen durch ständiges Abpumpen auf 0,4 l begrenzt. Nach Kühlen des Reaktoraustrags auf ca. 15°C wird das auskristallisierte Reaktionsprodukt von der zur Reaktion zurückzuführenden Mutterlauge abgetrennt, mit ca. 4 kg/h wäßrigem, 30 %igem Ethanol gewaschen und getrocknet. Unter Rückführung der Mutterlauge werden 4,5 kg/h 2'-Methylacetoacetanilid (Schmp. 105°C) entsprechend einer Ausbeute von 96,5 % bzgl. Amin erhalten.

### Beispiel 5

3,6 kg/h (19,2 mol/h) 4-Chlor-2,5-Dimethoxyanilin werden in 13,5 kg/h wäßriges 50 %iges Ethanol, von dem man in nachfolgenden Reaktionscyclen 11 kg/h durch anfallende Mutterlauge ersetzt, unter Erwärmen gelöst. Die ca. 80°C heiße Lösung wird unter gleichzeitiger Dosierung von 2 kg/h (23,3 mol/h) Diketen (Reinheit 98 %) in den Reaktor von Beispiel 1 eindosiert. Das heftig unter Rückfluß bei ca. 85°C siedende Reaktionsgemisch wird durch ständiges Abpumpen auf ein Volumen von ca. 0,4 l begrenzt. Der Reaktoraustrag wird gekühlt und unmittelbar vor Erreichen der Kristallisationstemperatur von ca. 55°C, mit geringen Mengen einer ca. 10 %igen wäßrigen Lösung von Dithionit versetzt, um eine spontane Aufhellung des rotbraunen Reaktoraustrags und somit helle Kristalle zu erzielen. Nach weiterem Kühlen bis auf ca. 15°C, Filtrieren des kristallinen Produkts von Mutterlauge, Wäsche mit 50 %igem wäßrigen Ethanol und Trocknung, werden unter der Bedingung der Mutterlaugenrückführung 5 kg/h (18,4 mol/h) grob kristallines 4'-Chlor-2',5'-dimethoxyacetoacetanilid (Schmp. 105-106°C) in einer Ausbeute von 96 % bzgl. Amin erhalten.

## Patentansprüche

1. Verfahren zur Herstellung von Acetoacetarylamiden der Formel I 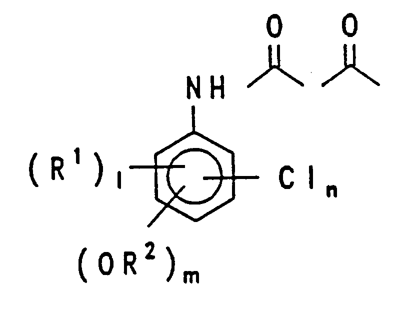 worin
R¹, R² gleiche oder verschiedene (C₁-C₃) Alkylreste
l, m = 0, 1 oder 2
n = 0 oder 1
sind,
durch Addition von Diketen an das entsprechende Arylamin, dadurch gekennzeichnet, daß man das Arylamin in Gegenwart eines Gemisches aus Wasser und eines (C₁-C₄)-Alkanols innerhalb von 0,1 bis 10 min. bei Temperaturen von 60°C bis 100°C kontinuierlich mit Diketen umsetzt.

2. Verfahren nach Anspruch 1, dadurch gekennzeichnet, daß man als Arylamin Anilin, 2-Methylanilin, 2,4-Dimethoxyanilin, 4-Isopropylanilin, 2-Methoxyanilin, 4-Ethoxyanilin, 2,4-Dimethylanilin, 2,5-Dimethoxyanilin, 2-Chloranilin oder 4-Chlor-2,5-dimethoxyanilin einsetzt.

3. Verfahren nach mindestens einem der Ansprüche 1 oder 2, dadurch gekennzeichnet, daß das Diketen im Überschuß von 3 bis 30 Mol-%, insbesondere 5 bis 25 Mol-%, eingesetzt wird.

4. Verfahren nach mindestens einem der Ansprüche 1 bis 3, dadurch gekennzeichnet, daß das Wasser/Alkohol-Gemisch einen Siedepunkt von 70°C bis 100°C aufweist.

5. Verfahren nach mindestens einem der Ansprüche 1 bis 4, dadurch gekennzeichnet, daß als Alkohol Methanol, Ethanol oder Propanol, insbesondere Ethanol eingesetzt wird.

6. Verfahren nach mindestens einem der Ansprüche 1 bis 5, dadurch gekennzeichnet, daß als Alkohol Ethanol eingesetzt wird und das Gemisch zwischen 20 und 80 Gew.-% Wasser enthält.

7. Verfahren nach mindestens einem der Ansprüche 1 bis 6, dadurch gekennzeichnet, daß das Gewichtsverhältnis Amin zu Alkohol/Wasser-Gemisch 1:1 bis 1:20, insbesondere 1:1 bis 1:15, bevorzugt 1:1 bis 1:10 beträgt.

8. Verfahren nach mindestens einem der Ansprüche 1 bis 7, dadurch gekennzeichnet, daß die Umsetzung innerhalb von 0,5 bis 5 Minuten, bevorzugt 0,75 bis 3 Minuten, durchgeführt wird.

9. Verfahren nach mindestens einem der Ansprüche 1 bis 8, dadurch gekennzeichnet, daß die Reaktionstemperatur 70 bis 95°C, insbesondere 75° bis 90°C, beträgt.

10. Verfahren nach mindestens einem der Ansprüche 1 bis 9, dadurch gekennzeichnet, daß das Arylamin und Diketen getrennt zudosiert werden.

11. Verfahren nach mindestens einem der Ansprüche 1 bis 10, dadurch gekennzeichnet, daß das Gemisch aus Alkohol und Wasser aus recyclisierter Mutterlauge besteht.

12. Verfahren nach mindestens einem der Ansprüche 1 bis 11, dadurch gekennzeichnet, daß man die bei der Umsetzung freiwerdende Wärme durch das Verdampfen des Gemischs von Alkohol und Wasser abführt.

13. Verfahren nach mindestens einem der Ansprüche 1 bis 12, dadurch gekennzeichnet, daß man das Verfahren bei Normaldruck, Überdruck oder Unterdruck, insbesondere Normaldruck durchführt.

## Claims

1. A process for preparing acetoacetarylamides of the formula I 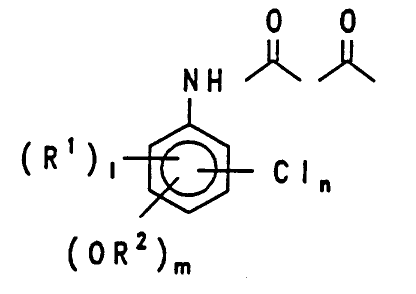 where
R¹ and R² are identical or different (C₁-C₃)-alkyl radicals,
l and m are each 0, 1 or 2, and
n is 0 or 1,
by addition of diketene to the appropriate arylamine, characterized in that the arylamine is continuously reacted with diketene in the presence of a mixture of water and of a (C₁-C₄)-alkanol at temperatures from 60°C to 100°C for from 0.1 to 10 min.

2. The process of claim 1, characterized in that the arylamine used is aniline, 2-methylaniline, 2,4-dimethoxyaniline, 4-isopropylaniline, 2-methoxyaniline, 4-ethoxyaniline, 2,4-dimethylaniline, 2,5-dimethoxyaniline, 2-chloroaniline or 4-chloro-2,5-dimethoxyaniline.

3. The process of at least one of claims 1 or 2, characterized in that the diketene is used in an excess of from 3 to 30 mol %, in particular from 5 to 25 mol %.

4. The process of at least one of claims 1 to 3, characterized in that the water-alcohol mixture has a boiling point from 70°C to 100°C.

5. The process of at least one of claims 1 to 4, characterized in that the alcohol used is methanol, ethanol or propanol, in particular ethanol.

6. The process of at least one of claims 1 to 5, characterized in that the alcohol used is ethanol and the mixture contains between 20 and 80 % by weight of water.

7. The process of at least one of claims 1 to 6, characterized in that the weight ratio of amine to alcohol-water mixture is from 1:1 to 1:20, in particular from 1:1 to 1:15, preferably from 1:1 to 1:10.

8. The process of at least one of claims 1 to 7, characterized in that the reaction is carried out in the course of from 0.5 to 5 minutes, preferably from 0.75 to 3 minutes.

9. The process of at least one of claims 1 to 8, characterized in that the reaction temperature is from 70 to 95°C, in particular from 75° to 90°C.

10. The process of at least one of claims 1 to 9, characterized in that the arylamine and diketene are metered separately.

11. The process of at least one of claims 1 to 10, characterized in that the mixture of alcohol and water is recycled mother liquor.

12. The process of at least one of claims 1 to 11, characterized in that the heat evolved in the course of the reaction is removed by evaporation of the mixture of alcohol and water.

13. The process of at least one of claims 1 to 12, characterized in that the process is carried out at atmospheric pressure, superatmospheric pressure or reduced pressure, in particular at atmospheric pressure.

## Revendications

1. Procédé pour la préparation d'acétoacétarylamides de formule I 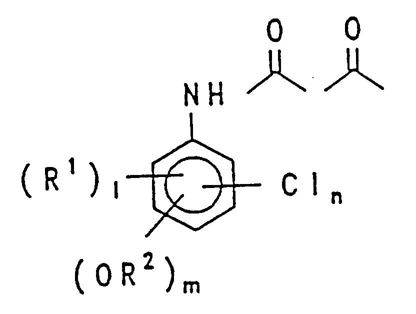 dans laquelle
R¹ et R² représentent des radicaux (C₁-C₃) alkyle idenitques ou différents
l, m = 0, 1 ou 2
n = 0 ou 1
par addition de dicétènes sur l'arylamine correspondante caractérisé en ce qu'on fait réagir en continu l'arylamine en présence d'un mélange d'eau et d'un alcanol en C₁-C₄, en l'espace de 0,1 à 10 minutes, à des températures de 60 à 100°C, avec le dicétène.

2. Procédé selon la revendication 1, caractérisé en ce qu'on utilise comme arylamine l'aniline, la 2-méthylaniline, la 2,4-diméthoxyaniline, la 4-ispropylaniline, la 2-méthoxyaniline, la 4-éthoxyaniline, la 2,4-diméthylaniline, la 2,5-diméthoxyaniline, la 2-chloraniline ou la 4-chloro-2,5-diméthoxyaniline.

3. Procédé selon au moins l'une des revendications 1 ou 2, caractérisé en ce qu'on utilise le dicétène en excès de 3 à 30% en moles, plus particulièrement de 5 à 25% en moles.

4. Procédé selon au moins l'une des revendications 1 ou 3, caractérisé en ce que le mélange eau/alcool présente un point d'ébullition de 70 à 100°C.

5. Procédé selon au moins l'une des revendications 1 ou 4, caractérisé en ce qu'on utilise comme alcool le méthanol, l'éthanol ou le propanol, plus particulièrement l'éthanol.

6. Procédé selon au moins l'une des revendications 1 ou 5, caractérisé en ce qu'on utilise l'éthanol en tant qu'alcool et le mélange contient une quantité d'eau comprise entre 20 et 80% en poids.

7. Procédé selon au moins l'une des revendications 1 ou 6, caractérisé en ce que le rapport pondéral de l'amine au mélange alcool/eau est de 1:1 à 1:20, notamment de 1:1 à 1:15, de préférence de 1:1 à 1:10.

8. Procédé selon au moins l'une des revendications 1 ou 7, caractérisé en ce qu'on met en oeuvre la réaction en l'espace de 0,5 à 5 minutes, de préférence de 0,75 à 3 minutes.

9. Procédé selon au moins l'une des revendications 1 ou 8, caractérisé en ce que la température de réaction est de 70 à 95°C, notamment de 75 à 90°C.

10. Procédé selon au moins l'une des revendications 1 ou 9, caractérisé en ce qu'on ajoute progressivement l'arylamine et le dicétène séparément.

11. Procédé selon au moins l'une des revendications 1 ou 10, caractérisé en ce que le mélange d'alcool et d'eau est constitué d'une liqueur-mère recyclable.

12. Procédé selon au moins l'une des revendications 1 ou 11, caractérisé en ce que la chaleur dégagée dans la réaction est évacuée par évaporation du mélange d'alcool et d'eau.

13. Procédé selon au moins l'une des revendications 1 ou 12, caractérisé en ce qu'on met en oeuvre le procédé à la pression normale, sous une pression élevée ou sous un défaut de pression, notamment à la pression normale.
